(19) Europäisches Patentamt

European Patent Office

Office européen des brevets

(11) Veröffentlichungsnummer: **0 283 833 B1**

# (12) EUROPÄISCHE PATENTSCHRIFT

(45) Veröffentlichungstag der Patentschrift: **06.05.92**

(21) Anmeldenummer: **88103580.2**

(22) Anmeldetag: **08.03.88**

(51) Int. Cl.⁵: **C07C 25/02**, C07C 17/38

(54) Verfahren zur Entfernung von m-Chlortoluol aus Chlortoluolgemischen.

(30) Priorität: **21.03.87 DE 3709415**

(43) Veröffentlichungstag der Anmeldung:
**28.09.88 Patentblatt 88/39**

(45) Bekanntmachung des Hinweises auf die
Patenterteilung:
**06.05.92 Patentblatt 92/19**

(84) Benannte Vertragsstaaten:
**DE FR GB IT**

(56) Entgegenhaltungen:
**EP-A- 0 099 161**
**EP-A- 0 220 664**
**US-A- 4 089 909**

(73) Patentinhaber: **BAYER AG**

**W-5090 Leverkusen 1 Bayerwerk(DE)**

(72) Erfinder: **Mais, Franz-Josef, Dr.**
**Kirchfeldstrasse 69**
**W-4000 Düsseldorf 1(DE)**
Erfinder: **Fiege, Helmut, Dr.**
**Walter-Flex-Strasse 23**
**W-5090 Leverkusen 1(DE)**

**Beschreibung**

Die Erfindung betrifft ein Verfahren zur Entfernung von m-Chlortoluol aus Chlortoluolgemischen mit einem Gehalt von bis zu 10 Gew.-% an m-Chlortoluol, bezogen auf die Gesamtmenge an Chlortoluol im Gemisch.

Chlortoluole sind in isomerenreiner Form wertvolle Zwischenprodukte für Pharmaka und Agrikulturchemikalien (Ullmanns Enzyklopädie der technischen Chemie, 4. Auflage, Band 9, Seite 514). Besonders im Bereich der Wirkstoffe ist die Anforderung an die Reinheit der Chlortoluole hoch.

Aus der US-PS 3 655 783 ist ein Verfahren zur Extraktion von m-Chlortoluol bekannt, wobei m-Chlortoluol aus einer Mischung von Chlortoluolen durch Zusatz von $HF/BF_3$ und einem organischen Lösungsmittel extrahiert wird. Die mit dem Zusatz der Fluorverbindungen verbundenen Nachteile, wie Einsatz teurer Werkstoffe zur Verhinderung von Korrosionsschäden und hoher apparativer Aufwand zur sicheren Handhabung der $HF/BF_3$-Mischung sind offensichtlich. Ebenso nachteilig ist der Zusatz eines Lösungsmittels, das die spätere Aufarbeitung durch Destillation erheblich erschwert.

Aus der EP-PS 0 099 161 ist ein Verfahren zur Trennung von gemischten Monochlortoluolen durch Adsorption an einer mit Zeolith gefüllten Säule und anschließende Desorption mit Readsorbentien bekannt. Nachteilig bei diesem Verfahren ist jedoch der sehr hohe technische Aufwand, mit dem die Trennung realisiert wird. Es werden zusätzliche Lösungsmittel, wie Toluol oder Chlorbenzol, benötigt, so daß zur Aufarbeitung des Gemisches zusätzliche Destillationen notwendig sind.

Es ist weiterhin bekannt (Ullmanns Enzyklopädie der technischen Chemie, 4. Auflage, Band 9, Seite 513), ein Gemisch aus den drei Chlortoluolisomeren zunächst fraktionierend zu trennen und das im p-Chlortoluol verbleibende m-Chlortoluol durch eine mehrstufige Schmelzkristallisation abzutrennen. Nachteilig sind der hohe technische Aufwand und die erheblichen Verluste an p-Chlortoluol, dem gegenüber o-Chlortoluol wesentlich wertvolleren Chlortoluolisomeren.

Es wurde nun ein Verfahren zur Entfernung von m-Chlortoluol aus Chlortoluolgemischen mit einem Gehalt von bis zu 10 Gew.-% an m-Chlortoluol, bezogen auf die Gesamtmenge an Chlortoluolen im Gemisch, gefunden, das dadurch gekennzeichnet ist, daß man ein weitgehend toluolfreies Chlortoluolgemisch in Gegenwart eines Friedel-Crafts-Katalysators und gegebenenfalls eines Co-Katalysators bei 0°C bis zum Siedepunkt des Gemisches chloriert, bis der Gehalt m-Chlortoluol auf einen Wert von <1,0 Gew.-% im Chlortoluolgemisch abgesunken ist.

Nach dem erfindungsgemäßen Verfahren soll der Gehalt an Toluol im Chlortoluolgemisch <2 Gew.-%, bevorzugt <1 Gew.-%, ganz besonders bevorzugt <0,5 Gew.-% betragen.

In das erfindungsgemäße Verfahren werden Chlortoluolgemische eingesetzt, die einen Gehalt an m-Chlortoluol von bis zu 10 Gew.-%, bezogen auf die Gesamtmenge an Chlortoluolen im Gemisch, besitzen. Besonders bevorzugt werden Chlortoluolgemische in das erfindungsgemäße Verfahren eingesetzt, die einen Gehalt von bis zu 2,5 Gew.-% an m-Chlortoluol, bezogen auf die Gesamtmenge an Chlortoluolen im Gemisch, aufweisen. Beispielsweise können Chlortoluolgemische in das erfindungsgemäße Verfahren eingesetzt werden, wie sie bei der Kernchlorierung von Toluol z.B. in flüssiger Phase in Gegenwart von Katalysatoren erhalten werden (vgl. Ullmanns Enzyklopädie der technischen Chemie, 4. Auflage, Band 9, Seite 510). Solche Chlortoluolgemische enthalten je nach Herstellungsweise etwa 0,3 bis 5 Gew.-% an m-Chlortoluol sowie etwa 70 bis 98 Gew.-% eines Gemisches aus o- und p-Chlortoluol, wobei das o/p-Verhältnis schwankt im Bereich von 3:1 bis 0,8:1 (o-:p-Chlortoluol). Der Rest besteht aus nicht umgesetztem Toluol sowie Di- und gegebenenfalls Trichlortoluolen.

Aus dem bei der Kernchlorierung erhaltenen Produktgemisch wird in üblicher Weise das Resttoluol abdestilliert. Zurück bleibt ein praktisch toluolfreier Sumpf, der je nach Herstellungsweise aus 0,5 bis 10 Gew.-% m-Chlortoluol, bis zu 98 Gew.-% eines Gemisches aus o- und p-Chlortoluol und einem Rest an höherchlorierten Chlortoluolen besteht.

Neben den oben beschriebenen Chlortoluolgemischen ist es nach dem erfindungsgemäßen Verfahren auch möglich, beliebige Gemische anderer Herkunft mit bis zu 10 Gew.-% an m-Chlortoluol, bezogen auf die Gesamtmenge an Chlortoluolen im Gemisch, einzusetzen.

Als Friedel-Crafts-Katalysatoren können in das erfindungsgemäße Verfahren folgende Verbindungen eingesetzt werden: Manganchloride, Molybdänchloride, Titanchloride, Eisen(III)-chlorid, Aluminiumchlorid, Zinkchlorid, Zinnchloride, Antimonchloride oder Gemische der genannten Verbindungen, bevorzugt sind Eisen(III)-chlorid, Aluminiumchlorid, Antimon(III)-chlorid, Antimon(V)-chlorid und/oder Zinkchlorid, ganz besonders bevorzugt Eisen(III)-chlorid. Die Menge an einzusetzendem Friedel-Crafts-Katalysator ist nicht kritisch und beträgt üblicherweise bis zu 5 Gew.-%, bevorzugt 0,001 bis 1 Gew.-%, bezogen auf die Menge an Chlortoluol.

EP 0 283 833 B1

Weiterhin können diesen Friedel-Crafts-Katalysatoren Co-Katalysatoren zugegeben werden. Genannt seien: Schwefel und/oder Schwefelverbindungen, z.B. Diphenylsulfid, Dischwefeldichlorid, Thianthren, Thianthrenderivate, Phenoxathiin, Phenoxathiinderivate, Phenothiazin und Phenothiazinderivate, sowie Iod und/oder Iodverbindungen. Bevorzugt sind die Co-Katalysatoren Schwefel, $S_2Cl_2$ und/oder Iod. Die Menge an Co-Katalysatoren beträgt üblicherweise 0,001 bis 5 Gew.-%, bevorzugt 0,01 bis 1 Gew.-%, bezogen auf die Gesamtmenge an Chlortoluol im Gemisch.

Erfindungsgemäß bevorzugt ist der Einsatz eines Friedel-Crafts-Katalysators mit einem Co-Katalysator. Die Verwendung von Co-Katalysatoren ist für das erfindungsgemäße Verfahren besonders günstig, da dabei die Bildung von höheren Chlortoluolen vermindert wird. Besonders die Weiterchlorierung des p-Chlortoluols, dem gegenüber o-Chlortoluol wesentlich wertvolleren Chlortoluolisomeren, wird reduziert im Vergleich mit der Chlorierung ohne Co-Katalysatoren.

Das Mengenverhältnis von Friedel-Crafts-Katalysator zu Co-Katalysator beträgt im allgemeinen 10:1 bis 1:10, bevorzugt 2:1 bis 1:2.

Die Chlorierung des Chlortoluolgemisches wird bei Temperaturen von etwa 0°C bis zum Siedepunkt des Gemisches, bevorzugt von 20 bis 80°C, besonders bevorzugt von 30 bis 50°C, durchgeführt.

Die erfindungsgemäße Chlorierung kann bei Normaldruck, Unterdruck oder Überdruck durchgeführt werden. Bevorzugt ist Normaldruck.

Erfindungsgemäß kann die Chlorierung mit elementarem Chlor in gasförmiger oder in flüssiger Form durchgeführt werden. Ebenso ist es möglich, äquivalente Mengen anderer Chlorierungsmittel zu verwenden, d.h. chlorhaltige Verbindungen, die unter den gewählten Reaktionsbedingungen das enthaltende Chlor in reaktiver Form abgeben können. Beispielsweise seien als solche Chlorierungsmittel genannt: Sulfurylchlorid, Chloroxide, wie Chlormonoxid, Chlorsulfonsäure und/oder Thionylchlorid. Erfindungsgemäß bevorzugt wird gasförmiges Chlor eingesetzt.

Nach dem erfindungsgemäßen Verfahren wird die Chlorierung des Chlortoluolgemisches soweit fortgeführt, bis der Gehalt an m-Chlortoluol auf einen Wert von <1,0 Gew.-%, bevorzugt <0,5 Gew.-%, besonders bevorzugt <0,1 Gew.-%, ganz besonders bevorzugt <0,05 Gew.-% im Chlortoluolgemisch abgesunken ist. Selbstverständlich ist es nach dem erfindungsgemäßen Verfahren auch möglich, die Chlorierung so zu steuern, bis ein höherer oder niederer Gehalt als die obengenannten an m-Chlortoluol im Chlortoluolgemisch sich eingestellt hat. Nach dem erfindungsgemäßen Verfahren ist es ausgesprochen einfach, den Fortgang der Verminderung der Menge an m-Chlortoluol z.B. durch gaschromatographische Analyse zu bestimmen und die Einleitung des Chlorierungsmittels abzubrechen, wenn der gewünschte niedrige Gehalt an m-Chlortoluol im Chlortoluolgemisch erreicht ist.

Das erfindungsgemäße Verfahren ist sowohl kontinuierlich als auch diskontinuierlich durchführbar.

Nach Beendigung der erfindungsgemäßen Chlorierung kann das o/p-Chlortoluolgemisch in üblicher Weise aufgearbeitet werden. Durch Destillation erhält man o- und p-Chlortoluolisomere von höchster Reinheit.

In einer besonders bevorzugten Ausführungsform des erfindungsgemäßen Verfahrens wird dem aus der Kernchlorierung von Toluol erhaltenen Chlortoluolgemisch, das nach dem Abdestillieren des Resttoluols noch den bei der Kernchlorierung zugesetzten Katalysator oder das Katalysatorgemisch und gegebenenfalls noch mindestens einen Co-Katalysator enthält, kein zusätzlicher Friedel-Crafts-Katalysator oder Co-Katalysator zugesetzt. Zur Katalyse der Chlorierung dienen dann die bereits im Chlortoluolgemisch enthaltenen Katalysatoren und gegebenenfalls die darin enthaltenen Co-Katalysatoren. Für die Ausführung des erfindungsgemäßen Verfahrens ist die Art des im Chlortoluolgemisch enthaltenen Katalysators und gegebenenfalls Co-Katalysators nicht kritisch, d.h. ein nach dem Stand der Technik für die Kernchlorierung von Toluol geeigneter Friedel-Crafts-Katalysator und gegebenenfalls Co-Katalysator ist für das vorliegende erfindungsgemäße Verfahren ebenfalls geeignet.

Es ist überraschend, daß bei dem erfindungsgemäßen Verfahren praktisch nur das m-Chlortoluol zu höheren Chlortoluolen aufchloriert wird und daß das o- und p-Chlortoluol unter diesen Bedingungen nicht wesentlich weiterchloriert wird, wobei vor allem an dem gegenüber o-Chlortoluol wertvolleren p-Chlortoluol kaum Verluste auftreten. Führt man das erfindungsgemäße Verfahren noch in Gegenwart von mindestens einem Co-Katalysator aus, so sind die Verluste an p-Chlortoluol noch geringer. Weiterhin ist überraschend, daß schon bei sehr geringer Überchlorierung der m-Chlortoluolgehalt im Chlortoluolgemisch praktisch verschwindet.

Das erfindungsgemäße Verfahren ist technisch besonders vorteilhaft, da es eine Abtrennung des m-Chlortoluols mit geringem Aufwand in einfachen Apparaturen ermöglicht. Ganz besonders vorteilhaft ist, daß das erfindungsgemäße Verfahren angewandt werden kann bei rohen Chlortoluolgemischen wie sie bei der Kernchlorierung von Toluol anfallen. Eine vorherige Reinigung des Chlortoluolgemisches ist dabei nicht mehr erforderlich. Die Trennung der Chlortoluolisomeren kann ohne großen Aufwand nach Durchführung des erfindungsgemäßen Verfahrens erfolgen.

Die nachfolgenden Beispiele sollen das erfindungsgemäße Verfahren verdeutlichen, ohne es jedoch auf diese Beispiele einzuschränken.

Beispiel 1

In einem Reaktor mit Rührer sowie Gasein- und -ableitung wurden 100 Gew.-Teile des Chlortoluolgemisches vorgelegt. Man gab die angegebenen Gewichtsteile an Katalysator und Co-Katalysator zu und leitete gasförmiges Chlor ein. Sobald man durch gaschromatographische Kontrolle einen gewünscht niedrigen Gehalt an m-Chlortoluol festgestellt hatte, brach man die Einleitung von Chlor ab,

Einsatzgemisch

51,55 % o-Chlortoluol (o-ClT)
0,40 % m-Chlortoluol (m-ClT)
47,94 % p-Chlortoluol (p-ClT)
0,11 % Dichlortoluole (DiClT)
0,025 Gew.-% $FeCl_3$
0,015 Gew.-% $S_2Cl_2$

| Temp. | $Cl_2$/mol % | o-ClT | m-ClT | p-ClT | DiClT |
|-------|---------|-------|-------|-------|-------|
| 27°C | 1,06 | 51,23 | 0,28 | 47,77 | 0,72 |
| 31°C | 3,33 | 50,39 | 0,12 | 47,39 | 2,10 |
| 32°C | 6,70 | 49,09 | 0,02 | 46,90 | 3,99 |

Die folgenden Beispiele 2 - 6 wurden entsprechend der Vorschrift des Beispiels 1 ausgeführt. Bei den Beispielen sind jeweils die Zusammensetzung des Chlortoluoleinsatzgemisches und die Menge und Art des Katalysators und des Co-Katalysators angegeben.

Beispiel 2

Einsatzgemisch

48,66 % o-Chlortoluol
1,74 % m-Chlortoluol
49,57 % p-Chlortoluol
0,03 % Dichlortoluole
0,015 Gew.-% $FeCl_3$
0,015 Gew.-% $S_2Cl_2$

| Temp. | $Cl_2$/mol % | o-ClT | m-ClT | p-ClT | DiClT |
|-------|---------|-------|-------|-------|-------|
| 30°C | 3,33 | 47,81 | 0,69 | 48,99 | 2,54 |
| 32°C | 6,62 | 46,21 | 0,19 | 48,34 | 5,26 |
| 33°C | 11,75 | 44,29 | 0,02 | 47,19 | 8,50 |

Beispiel 3

Einsatzgemisch

49,12 % o-Chlortoluol
0,93 % m-Chlortoluol
49,87 % p-Chlortoluol

0,04 % Dichlortoluole
0,015 Gew.-% FeCl$_3$
0,015 Gew.-% S$_2$Cl$_2$

| Temp. | Cl$_2$/mol % | o-ClT | m-ClT | p-ClT | DiClT |
|-------|-----------|-------|-------|-------|-------|
| 30°C | 3,45 | 47,97 | 0,28 | 49,16 | 2,59 |
| 32°C | 6,90 | 46,61 | 0,08 | 48,55 | 4,76 |
| 33°C | 10,11 | 45,15 | 0,01 | 47,80 | 7,04 |

Beispiel 4

Einsatzgemisch

49,34 % o-Chlortoluol
0,50 % m-Chlortoluol
50,10 % p-Chlortoluol
0,06 % Dichlortoluole
0,015 Gew.-% FeCl$_3$
0,015 Gew.-% S$_2$Cl$_2$

| Temp. | Cl$_2$/mol % | o-ClT | m-ClT | p-ClT | DiClT |
|-------|-----------|-------|-------|-------|-------|
| 32°C | 3,41 | 47,22 | 0,11 | 49,26 | 3,41 |
| 33°C | 6,75 | 46,13 | 0,02 | 48,50 | 5,35 |

Beispiel 5

Einsatzgemisch

0,21 % o-Chlortoluol
0,72 % m-Chlortoluol
99,07 % p-Chlortoluol
0,030 Gew.-% FeCl$_3$
0,015 Gew.-% Schwefel

| Temp. | Cl$_2$/mol % | Tol | o-ClT | m-ClT | p-ClT | DiClT |
|-------|-----------|-----|-------|-------|-------|-------|
| 30°C | 3,39 | - | 0,21 | 0,20 | 97,23 | 2,36 |
| 32°C | 6,77 | - | 0,20 | 0,03 | 95,04 | 4,73 |

Beispiel 6

Einsatzgemisch

76,16 % o-Chlortoluol
0,64 % m-Chlortoluol
20,18 % p-Chlortoluol
0,02 % Dichlortoluole
0,030 Gew.-% SbCl$_3$
0,015 Gew.-% S$_2$Cl$_2$

5

| Temp. | Cl$_2$/mol % | o-ClT | m-ClT | p-ClT | DiClT |
|-------|--------------|-------|-------|-------|-------|
| 40°C | 6,58 | 75,55 | 0,03 | 19,68 | 4,74 |

Beispiel 7

In einem Reaktor mit Rührer sowie Gasein- und -ableitung wurden 100 Gew.-Teile des Chlortoluolgemisches vorgelegt. Man gab die angegebene Menge an Katalysator zu und leitete gasförmiges Chlor ein, bis nach der GC-Analyse der gewünschte Gehalt an m-Chlortoluol sich eingestellt hatte.

Einsatzgemisch:

49,43 % o-Chlortoluol
0,47 % m-Chlortoluol
50,10 % p-Chlortoluol
0,025 Gew.-% FeCl$_3$

| Temp. | Cl$_2$/mol % | o-ClT | m-ClT | p-ClT | DiClT |
|-------|--------------|-------|-------|-------|-------|
| 27°C | 4,59 | 49,05 | 0,35 | 49,79 | 0,81 |
| 30°C | 9,22 | 47,41 | 0,13 | 48,54 | 3,92 |
| 31,5°C | 11,51 | 46,28 | 0,03 | 47,84 | 5,85 |

Beispiel 8

Man legte in einem Reaktor unter Rühren 100 Gew.-Teile Toluol vor und setzte die angegebenen Mengen Katalysator und Co-Katalysator zu. Danach wurde, wie nach dem Stand der Technik üblich, das Toluol zu einem Gemisch aus Toluol, isomeren Chlortoluolen und höheren Chlortoluolen aufchloriert, Nachdem das Resttoluol durch eine technisch übliche Destillation entfernt wurde, leitete man in das Gemisch der angegebenen Zusammensetzung bei der angegebenen Temperatur solange Chlor gasförmig ein, bis nach der GC-Analyse der gewünschte Gehalt an m-Chlortoluol sich eingestellt hatte,

Einsatzgemisch

0,10 % Toluol
51,05 % o-Chlortoluol
0,27 % m-Chlortoluol
46,58 % p-Chlortoluol
2,00 % Dichlortoluole
Restkatalysator der Kernchlorierung
ca. 0,01 Gew.-% FeCl$_3$
ca. 0,01 Gew,-% Schwefel

| Temp. | Cl$_2$/mol % | Toluol | o-ClT | m-ClT | p-ClT | DiClT |
|-------|--------------|--------|-------|-------|-------|-------|
| 36°C | 3,18 | 0,03 | 50,37 | 0,12 | 46,31 | 3,17 |
| 36°C | 5,54 | 0,01 | 48,50 | 0,01 | 45,76 | 5,72 |

Das Beispiel 9 wurde wie Beispiel 8 ausgeführt, nur daß nach dem Stand der Technik ein anderer Katalysator und ein anderer Co-Katalysator verwendet wurden.

6

EP 0 283 833 B1

Beispiel 9

Einsatzgemisch

0,14 % Toluol
45,48 % o-Chlortoluol
0,43 % m-Chlortoluol
53,49 % p-Chlortoluol
0,36 % Dichlortoluole
Restkatalysator aus der Kernchlorierung
0,100 Gew.-% 2,7-Dichlorthianthren
0,027 Gew.-% $SbCl_3$

| Temp. | $Cl_2$/mol % | Toluol | o-ClT | m-ClT | p-ClT | DiClT |
|-------|--------------|--------|-------|-------|-------|-------|
| 50 °C | 3,39 | 0,02 | 44,73 | 0,19 | 53,21 | 1,87 |
| 50 °C | 8,63 | - | 42,19 | 0,02 | 52,14 | 5,65 |

Beispiel 10

In einen Reaktor mit Rührer und Gasableitung wurden 100 Gew.-Teile des Chlortoluolgemisches vorgelegt. Man gab die angegebenen Gewichtsteile an Katalysator und Co-Katalysator zu und leitete 13 Mol-% Sulfurylchlorid flüssig ein, Man rührte 12 h bei der angegebenen Temperatur und rührte die Reaktionsmischung mit dem gleichen Volumen Wasser aus,

Einsatzgemisch

49,40 % o-Chlortoluol
0,46 % m-Chlortoluol
50,14 % p-Chlortoluol
0,07 Gew.-% $FeCl_3$
0,07 Gew.-% Schwefel

| Temp. | o-ClT | m-ClT | p-ClT | DiClT |
|-------|-------|-------|-------|-------|
| 40 °C | 46,03 | 0,02 | 48,82 | 5,13 |

## Patentansprüche

1. Verfahren zur Entfernung von m-Chlortoluol aus Chlortoluolgemischen mit einem Gehalt von bis zu 10 Gew.-% an m-Chlortoluol, bezogen auf die Gesamtmenge an Chlortoluolen im Gemisch, dadurch gekennzeichnet, daß man ein weitgehend toluolfreies Chlortoluolgemisch in Gegenwart eines Friedel-Crafts-Katalysators und gegebenenfalls eines Co-Katalysators bei 0 °C bis zum Siedepunkt des Gemisches chloriert, bis der Gehalt an m-Chlortoluol auf einen Wert von <1,0 Gew.-% im Chlortoluolgemisch abgesunken ist.

2. Verfahren nach Anspruch 1, dadurch gekennzeichnet, daß der Toluolgehalt im Chlortoluolgemisch kleiner als 2 Gew.-% ist.

3. Verfahren nach Anspruch 1 und 2, dadurch gekennzeichnet, daß als Friedel-Crafts-Katalysatoren Eisen-(III)-chlorid, Aluminiumchlorid, Antimon(III)-chlorid, Antimon(V)-chlorid und/oder Zinkchlorid eingesetzt werden.

4. Verfahren nach Ansprüchen 1 bis 3, dadurch gekennzeichnet, daß als Co-Katalysatoren Schwefel, Schwefelverbindungen, Iod und/oder Iodverbindungen eingesetzt werden.

7

**5.** Verfahren nach Ansprüchen 1 bis 4, dadurch gekennzeichnet, daß als Schwefelverbindungen Dischwefeldichlorid, Diphenylsulfid, Thianthren, Thianthrenderivate, Phenoxathiin, Phenoxathiinderivate, Phenothiazin und/oder Phenothiazinderivate eingesetzt werden.

**6.** Verfahren nach Ansprüchen 1 bis 5, dadurch gekennzeichnet, daß man die Chlorierung mit gasförmigem oder flüssigem Chlor oder einer äquivalenten Menge eines anderen Chlorierungsmittels durchführt.

**7.** Verfahren nach Ansprüchen 1 bis 6, dadurch gekennzeichnet, daß man als Chlorierungsmittel Sulfurylchlorid verwendet.

**8.** Verfahren nach Ansprüchen 1 bis 7, dadurch gekennzeichnet, daß man rohe, weitgehend toluolfreie Chlortoluolgemische aus der Chlortoluolherstellung einsetzt, die noch den Friedel-Crafts-Katalysator und gegebenenfalls den Co-Katalysator enthalten.

**9.** Verfahren nach Ansprüchen 1 bis 8, dadurch gekennzeichnet, daß man die Chlorierung bei Temperaturen von 20 bis 80°C durchführt.

**Claims**

**1.** Process for the removal of m-chlorotoluene from chlorotoluene mixtures having a content of up to 10% by weight of m-chlorotoluene, with reference to the total amount of chlorotoluenes in the mixture, characterized in that a substantially toluene-free chlorotoluene mixture is chlorinated in the presence of a Friedel-Crafts catalyst and if appropriate a co-catalyst at from 0°C up to the boiling point of the mixture, until the content of m-chlorotoluene has decreased to a value of <1.0 % by weight in the chlorotoluene mixture.

**2.** Process according to Claim 1, characterized in that the toluene content in the chlorotoluene mixture is less than 2 % by weight.

**3.** Process according to Claim 1 and 2, characterized in that iron (III) chloride, aluminium chloride, antimony (III) chloride, antimony(V) chloride and/or zinc chloride are employed as Friedel-Crafts catalysts.

**4.** Process according to Claims 1 to 3, characterized in that sulphur, sulphur compounds, iodine and/or iodine compounds are employed as co-catalysts.

**5.** Process according to Claims 1 to 4, characterized in that disulphur dichloride, diphenyl sulphide, thianthrene, thianthrene derivatives, phenoxathiin, phenoxathiin derivatives, phenothiazine and/or phenothiazine derivatives are employed as sulphur compounds.

**6.** Process according to Claims 1 to 5, characterized in that the chlorination is carried out with gaseous or liquid chlorine or an equivalent amount of another chlorinating agent.

**7.** Process according to Claims 1 to 6, characterized in that sulphuryl chloride is used as chlorinating agent.

**8.** Process according to Claims 1 to 7, characterized in that crude, substantially toluene-free chlorotoluene mixtures from the chlorotoluene preparation are employed which still contain the Friedel-Crafts catalyst and if appropriate the co-catalyst.

**9.** Process according to Claims 1 to 8, characterized in that the chlorination is carried out at temperatures of 20 to 80°C.

**Revendications**

1. Procédé d'élimination du m-chlorotoluène de mélanges de chlorotoluènes ayant une teneur maximale en m-chlorotoluène de 10 % en poids, par rapport à la quantité totale de chlorotoluènes dans le mélange, caractérisé en ce qu'on chlore un mélange de chlorotoluènes sensiblement exempt de toluène en présence d'un catalyseur de Friedel-Crafts et le cas échéant d'un co-catalyseur à une température comprise entre 0°C et le point d'ébullition du mélange, jusqu'à ce que la teneur en m-chlorotoluène ait été ramenée à un chiffre inférieur à 1,0 % en poids dans le mélange de chlorotoluènes.

2. Procédé selon la revendication 1, caractérisé en ce que la teneur en toluène dans le mélange de chlorotoluènes est inférieure à 2 % en poids.

3. Procédé selon les revendications 1 et 2, caractérisé en ce qu'on utilise comme catalyseurs de Friedel-Crafts du chlorure de fer(III), du chlorure d'aluminium, du chlorure d'antimoine(III), du chlorure d'antimoine(V) et/ou du chlorure de zinc.

4. Procédé selon les revendications 1 à 3, caractérisé en ce qu'on utilise comme co-catalyseurs du soufre, des composés soufrés, de l'iode et/ou des composés iodés.

5. Procédé selon les revendications 1 à 4, caractérisé en ce qu'on utilise comme composés soufrés du dichlorure de di-soufre, du sulfure de diphényle, du thianthrène, des dérivés de thianthrène, de la phénoxathiine, des dérivés de la phénoxathiine, de la phénothiazine et/ou des dérivés de la phénothiazine.

6. Procédé selon les revendications 1 à 5, caractérisé en ce qu'on procède à la chloration à l'aide de chlore gazeux ou liquide ou d'une quantité équivalente d'un autre agent de chloration.

7. Procédé selon les revendications 1 à 6, caractérisé en ce qu'on utilise comme agent de chloration du chlorure de sulfuryle.

8. Procédé selon les revendications 1 à 7, caractérisé en ce qu'on utilise des mélanges de chlorotoluènes bruts, pratiquement exempts de toluène provenant de la fabrication de chlorotoluène, contenant encore le catalyseur de Friedel-Crafts et le cas échéant le co-catalyseur.

9. Procédé selon les revendications 1 à 8, caractérisé en ce qu'on procède à la chloration à des températures comprises entre 20 et 80°C.